# EUROPEAN PATENT SPECIFICATION

(11) **EP 2 392 287 B1**
(45) Date of publication and mention of the grant of the patent: **09.12.2020**
(21) Application number: 10748518.7
(22) Date of filing: 03.03.2010
(51) Int. Cl.: A61C 3/02, A61B 18/20, A61C 1/00, A61C 17/02, A61B 18/22

(54) **DENTAL PROBE**
ZAHNÄRZTLICHE SONDE
SONDE DENTAIRE

(30) Priority: 05.03.2009 JP 2009052093
(43) Date of publication of application: 07.12.2011
(73) Proprietor: Fujikura Ltd., Tokyo 135-8512 (JP)
(72) Inventor: NAKATATE, Kenichi, Sakura-shi Chiba 285-8550 (JP); HU, Wei-Zhi, Sakura-shi Chiba 285-8550 (JP)
(74) Representative: Plasseraud IP
(86) International application number: PCT/JP2010/001464
(87) International publication number: WO 2010/100919

(56) References cited:
- EP-A1- 1 930 752
- JP-A- 6 258 531
- JP-A- 6 258 531
- JP-A- 10 211 211
- JP-A- 11 188 107
- JP-A- 55 117 104
- JP-A- H05 344 982
- JP-A- H07 190 864
- JP-B- 55 020 562
- JP-B2- 3 124 643

## Description

### TECHNICAL FIELD

The present invention relates to a dental probe which is provided in a handpiece for dental treatments and used for guiding laser light transmitted through an optical fiber to a position to be irradiated.

### BACKGROUND ART

Recently, a dental laser treatment device used for dental hard tissue treatments such as the removal of dental caries and dentin and the excision of enamel has been provided.

A laser handpiece used for the dental laser treatment device includes a tip portion guiding laser light emitted from an optical fiber to a treatment site (a position to be irradiated).

It is preferable that the tip portion possess a shape and a structure fit for the treatment site of the teeth and the mode of treatment and can remove evaporated substances by cooling an affected area irradiated with the laser light.

Accordingly, it is known that, in a laser handpiece including a probe guiding the laser light transmitted through the optical fiber to the position to be irradiated, the probe includes a water injection tube injecting water near the exit end of the probe and a gas supply tube injecting gas (air) near the exit end of the probe, and is so formed that the shape of the tip portion of the probe causes the direction of the laser light emitted from the optical fiber to incline with respect to the axis of the laser handpiece (see Patent Document 1,for example).

Further, document JP H05 344982 A focuses on how to permit the laser beam radiation from the top edge of a handpiece and the injection of cleaning water, etc., and prevent a light introducing fiber from being given with the influence of moistre, by forming a light introducing built-in part airtight.

### PRIOR ART DOCUMENTS

### PATENT DOCUMENTS

[Patent Document 1] Japanese Patent No. 3124643
[Patent Document 2] Japanese patent publication JP H05 344982 A

### DISCLOSURE OF INVENTION

### PROBLEMS TO BE SOLVED BY THE INVENTION

However, from the probe for dental treatment, a powerful laser light is emitted to remove the dental hard tissue.

On the other hand, there is a problem in that, since the tip portion of the optical fiber bends in the tip portion of the probe, a portion of the laser light leaks out of the optical fiber due to the bending loss of the optical fiber, and the leaking power is converted to heat, whereby the temperature of the probe tip portion rises.

When the temperature of the probe tip portion rises, there is a concern that a patient will experience unpleasant sensations since he or she can feel the intense heat of the probe tip portion.

When the temperature of the probe tip portion rises remarkably, there is a concern that a burn will be caused by contact of the lateral surface of the probe tip portion to the oral cavity, the gums, and the like.

The present invention has been made under the consideration of the above circumstances, and provides a dental probe that can prevent a temperature rise in the tip portion.

### MEANS FOR SOLVING THE PROBLEMS

In order to solve the above problems, the present invention provides a dental probe provided in a laser handpiece for guiding laser light transmitted through an optical fiber to a position to be irradiated during dental treatment as defined by claim 1. A corresponding method for manufacturing a dental probe as defined in claim 3 is also provided.

Preferred embodiments of the dental probe according to claim 1 an the method for manufacturing a dental probe according to claim 3 are defined by the dependent claims.

### EFFECTS OF THE INVENTION

According to the invention, the resin-coated layer is formed of a polymer compound having a refractive index lower than that of the cladding.

Accordingly, the resin-coated layer can function as a second cladding in the optical fiber to suppress the leakage of the laser light caused by the bending loss.

As a result, it is possible to prevent a temperature rise in the probe.

### BRIEF DESCRIPTION OF THE DRAWINGS

FIG. 1A is a front view illustrating an example of a dental probe of the invention.
FIG. 1B is a rear view of a portion taken excluding a tip portion of the dental probe.
FIG. 1C is a view illustrating the tip portion of the dental probe, which is a cross sectional view taken along the line S-S in FIG. 1A.
FIG. 2 is a cross-sectional view taken along the axial direction of the dental probe illustrated in FIGS. 1A to 1C.
FIG. 3 is a schematic view illustrating a configuration example of a laser handpiece including the dental probe illustrated in FIGS. 1A to 1C.

### EMBODIMENTS FOR CARRYING OUT THE INVENTION

Hereinafter, based on preferable embodiments, the invention will be described with reference to drawings.

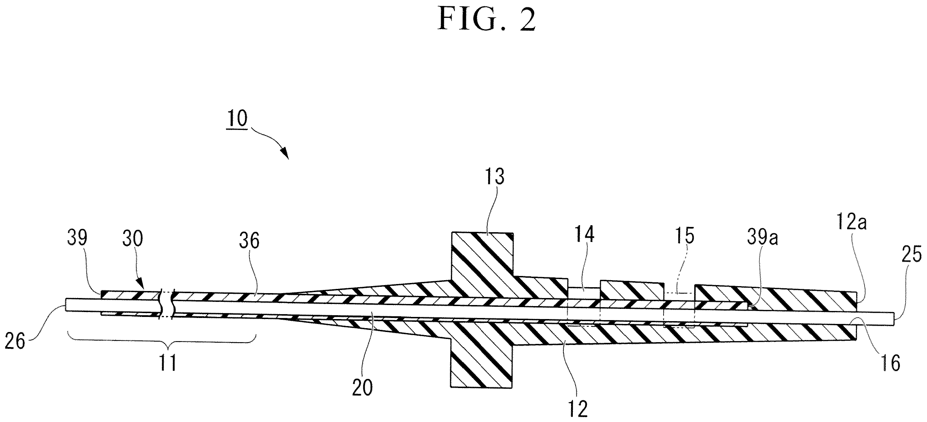

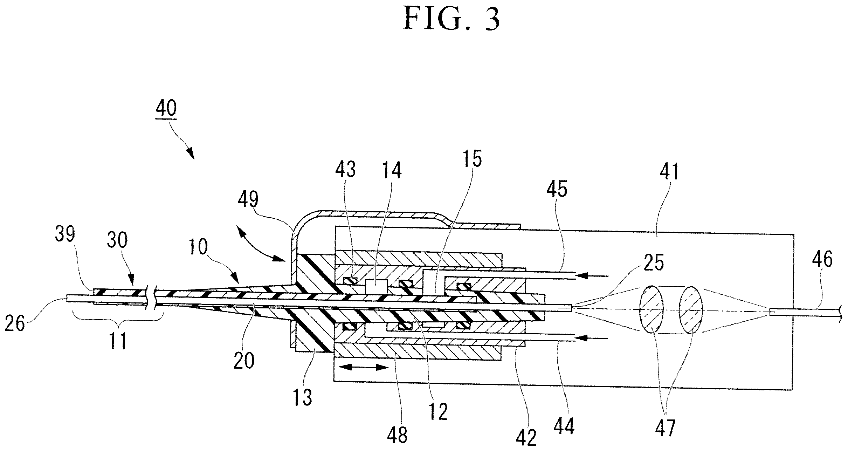

FIGS. 2 and 3 illustrate the dental probe by omitting the middle portion of a probe tip portion 11.

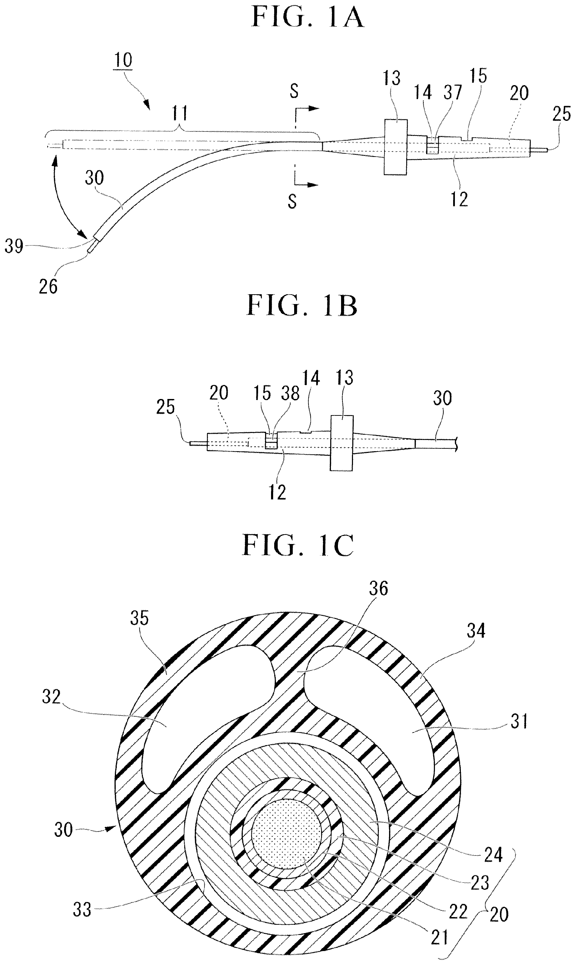

As shown in FIGS. 1A to 1C, the tip portion 11 of a dental probe 10 of the embodiment includes a water flow path 31, an air flow path 32, a multi-lumen tube 30 having a duct 33 formed therein in which an optical fiber 20 is inserted, and the optical fiber 20 inserted into the duct 33; the optical fiber 20 includes a core 21, a cladding 22, a resin-coated layer 23 provided on the outer circumference of the cladding 22, a metal-coated layer 24 provided on the outer circumference of the resin-coated layer 23; and the resin-coated layer 23 is formed of a polymer compound having a refraction index lower than that of the cladding 22.

The core 21 and the cladding 22 of the optical fiber 20 can be configured with various optical fibers such as a silica-based optical fiber, a polymer clad optical fiber, a fluoride optical fiber, a chalcogenide glass optical fiber, and the like.

In the embodiment, a large diameter optical fiber in which a core diameter thereof (for example, a core diameter of 400 µm) is higher than that of an ordinary optical fiber for communication is used.

In the invention, not only the large diameter optical fiber, but optical fibers for energy transmission such as an image fiber (a multi-core optical fiber) and a polymer clad fiber can be used, and the types and the structures of the fibers are not particularly limited.

The resin-coated layer 23 is formed of a polymer compound having a refractive index lower than that of the cladding 22.

The material of the polymer compound used for the resin-coated layer 23 is selected according to the refractive index of the cladding 22.

For example, when the cladding 22 is formed of a pure silica glass, examples of the polymer compound having a refractive index lower than that (about 1.463) of the pure silica glass include a silicon resin, a fluoro-acrylic resin, a vinyl acetate resin, and the like.

In comparison, polymethyl methacrylate, polyimide, polycarbonate, and the like have a refractive index higher than that of the pure silica glass.

Even when a resin has a refractive index higher than that of the pure silica glass, if the refractive index of the core and the cladding is higher than that of the resin, the resin can be used for the invention.

It is preferable that the refractive index of the resin-coated layer 23 be 0.04 or more lower than that of the pure silica.

The metal-coated layer 24 can be configured with a metal such as copper (Cu), tin (Sn), or nickel (Ni) or with an alloy such as a Cu alloy, a Sn alloy, or Ni alloy.

When the metal-coated layer 24 is prepared by electrolytic plating, in order to impart conductivity to the surface of the resin-coated layer 23, a base metal layer is formed by a method such as electroless plating, sputtering, vacuum deposition, a chemical vapor deposition method, or the like.

It is preferable that the metal-coated layer 24 have a sufficient thickness that can hold the shape of the optical fiber 20 which is provided while bending in the probe tip portion 11 as shown in FIG. 1A.

Furthermore, it is preferable that the metal-coated layer 24 be provided with such a thickness that the optical fiber 20 has a plasticity (deformability) so that the probe tip portion 11 can be stretched linearly or bent or curved into a desired shape as shown by the two-dot chain line in FIG. 1A.

Generally, the probe includes a metal-coated layer; therefore, the probe is configured so that, even when the leakage of light is assumed to be caused by bending of the probe, the light does not leak out of the probe.

As shown in FIG. 1C, the multi-lumen tube 30 includes the duct 33 in which the water flow path 31, the air flow path 32, and the optical fiber 20 are inserted.

The optical fiber 20 is inserted into the duct 33.

One end of the multi-lumen tube 30 is a tip surface 39.

The other end of the multi-lumen tube 30 is housed within a probe body portion 12, as shown in FIGS. 1A to 1B and FIG. 2.

The shape of the exit end 26 of the optical fiber 20 is not limited to a planar shape perpendicular to the optical axis.

The shape can also be formed (or polished for example) into a bevel or a cone.

In the embodiment, the probe tip portion 11 is formed of the multi-lumen tube 30 and the optical fiber 20.

The multi-lumen tube 30 can be configured with a polymer compound such as urethane, stainless steel, or the like.

The multi-lumen tube 30 is formed of a polymer compound having flexibility, since the probe tip portion 11 can be deformed into a desired shape using one's fingers.

In the probe 10 of the embodiment, the metal-coated layer 24 is provided on the optical fiber 20.

Therefore, even when the probe tip portion 11 is bent, or the bending shape thereof is arbitrarily changed (deformed), it is possible to reduce the stress applied to the optical fiber 20 to suppress the damage or breaking of the optical fiber 20.

As shown in FIGS. 1C and 2, the duct 33 in which the optical fiber 20 is inserted is formed to continue from the tip surface 39 of the multi-lumen tube 30 to an end surface 39a at the other side.

In FIG. 1C, a clearance is shown between the duct 33 and the optical fiber 20; however, the duct 33 and the optical fiber 20 may be close to each other to such a degree that the optical fiber 20 can be inserted into the duct 33 without difficulty.

The water flow path 31 is partitioned from the air flow path 32 by a partition wall 36 throughout the entire length of the tube.

The water flow path 31 and the air flow path 32 are opened in the tip surface 39 of the multi-lumen tube 30.

When laser light is emitted from the exit end 26 of the optical fiber 20, water and air are respectively ejected from openings (outlets) of the flow paths 31 and 32, whereby the evaporated substances can be removed by cooling the position to be irradiated.

The probe body portion 12 can be configured with various materials such as polymer compounds (a resin, rubber, or the like) or metals, and the structure thereof is not particularly limited.

In the embodiment, the probe body portion 12 is made of a polymer compound and is formed integrally with the multi-lumen tube 30 made of a polymer compound.

Notches 14 and 15 are provided on the lateral surface of the probe body portion 12.

The multi-lumen tube 30 is exposed through each of the notches 14 and 15, and thin portions 34 and 35 of the multi-lumen tube 30 are cut and opened to form an inlet 37 of the water flow path 31 and an inlet 38 of the air flow path 32.

In the end surface 39a at the opposite to the tip surface 39 of the multi-lumen tube 30, the water flow path 31 and the air flow path 32 are closed in the probe body portion 12.

An incident end 25 of the optical fiber 20 extends backwardly (the right side of FIG. 2) from the end surface 39a of the multi-lumen tube 30, passes through an optical fiber path 16 that is in the probe body portion 12, and protrudes from a rear end surface 12a of the probe body portion 12.

When the probe body portion 12 is manufactured by insert molding, the flow paths 31 and 32 are filled with a detachable reinforcement material, the multi-lumen tube 30 including the duct 33 in which the optical fiber 20 is inserted is prepared, and a molten resin is supplied to the circumference of the optical fiber 20 and the multi-lumen tube 30 and solidified, whereby the optical fiber path 16 can be formed by using the shape of the exterior surface of the optical fiber 20 as a mold.

At this time, the notches 14 and 15 leading to the inlets 37 and 38 do not need to be opened in post-processing of the probe body portion 12.

The notches can also be formed by, for example, providing fins closing the inlets 37 and 38 to the interior surface of a mold so that the molten resin is not provided to the portions of the inlets 14 and 15.

In a laser handpiece for dental treatments, the dental probe 10 of the embodiment can be used for guiding laser light transmitted through the optical fiber 20 to a position to be irradiated.

FIG. 3 illustrates a configuration example of a laser handpiece.

A handpiece 40 herein is configured so that for example, an operator (particularly, a dentist) can operate the handpiece by gripping it with his or her hand, and the dental probe 10 is detachably mounted on a receptacle 42 provided at the tip side of a handpiece body 41.

The receptacle 42 presses the outer circumferential surface of the probe body portion 12 in the direction facing the central axis thereof with an O-ring 43, for example, and stabilizes the position of the optical axis.

Furthermore, by engaging the fastener 49 with a flange portion 13 which is formed to protrude from the exterior surface of the probe body portion 12, it is possible to prevent the dental probe 10 from coming off accidentally in use.

When the dental probe 10 is detached from the handpiece body 41, the fastener 49 is lifted up, an ejector 48 is moved to the tip side (the left side in FIG. 3) of the handpiece body 41 by using operation means (not shown), therefore the dental probe 10 is pushed out of the receptacle 42.

The handpiece body 41 is provided with a water supply tube 44 and an air supply tube 45.

The water supply tube 44 and the air supply tube 45 are connected to water and air supply devices (not shown).

The water supply tube 44 is connected to a water inlet 37 (see FIG. 1A) provided in the notch 14 of the probe body portion 12.

The air supply tube 45 is connected to an air inlet 38 (see FIG. 1B) provided in the notch 15.

At the rear end side of the handpiece body 41, an optical fiber 46 connected to a laser light source for supplying laser light is held.

The optical fiber 46 at the light source side and the optical fiber 20 of the probe 10 are optically coupled with each other in a non-contact manner through a collimator lens 47.

Consequently, compared to a case where the end surfaces of the optical fibers 20 and 46 are connected by being brought into contact with each other, an external force is not applied to the end surfaces of the optical fibers 20 and 46 in mounting or detaching the probe 10, so it is possible to prevent the end surfaces of the optical fibers 20 and 46 from being damaged.

During the dental treatment, the operator configures the handpiece 40 by mounting the probe 10 having an appropriate tip shape on the handpiece body 41, and emits laser light to a lesion while supplying water and air.

In this manner, the operator can perform the dental tissue treatment.

Even when the probe tip portion 11 is bent into a desired shape, a temperature rise of the probe tip portion 11 caused by the leakage of light from the optical fiber 20 is suppressed.

Therefore, there is no concern that a patient will experience an unpleasant sensation since he or she can feel the intense heat of the probe tip portion 11 or that the patient will be burned.

### Examples

The present invention will be described in more detail based on examples.

The invention is not based only on the shape and dimensions of the examples, and is not particularly limited to the shape and dimensions including the core diameter, cladding diameter, fiber length, bending angle, coating diameter, metal coating diameter, tube structure as well as the material thereof.

<Example 1> As shown in FIG. 1C, a metal-coated optical fiber was used which was obtained by coating copper having a thickness of 50 µm on the outside of a resin layer which was formed of a silicon resin (refractive index; 1.35) having a thickness of 50 µm on the outside of a large diameter fiber (core diameter; ϕ400 µm, cladding diameter; ϕ500 µm).

The metal-coated optical fiber had a structure in which a length thereof was 40 mm, and a bending angle at the central portion thereof was 30 °.

This fiber was inserted into a tube made of urethane having a multi-lumen structure, followed by integral molding by using a mold, therefore preparing a probe.

<Example 2> A probe was prepared in the same manner as in Example 1, except that a fluoro-acrylic resin (refractive index; 1.42) was used instead of the silicon resin.

<Example 3> A probe was prepared in the same manner as in Example 1, except that a vinyl acetate resin (refractive index; 1.46) was used instead of the silicon resin.

<Comparative example 1> A probe was prepared in the same manner as in Example 1, except that polymethyl methacrylate (refractive index; 1.49) was used instead of the silicon resin.

<Comparative example 2> A probe was prepared in the same manner as in Example 1, except that a polyimide resin (refractive index; 1.52) was used instead of the silicon resin.

<Comparative example 3> A probe was prepared in the same manner as in Example 1, except that a polycarbonate resin (refractive index; 1.59) was used instead of the silicon resin.

<Test example> The samples (n = 10) of the examples and comparative examples were continuously irradiated for 10 seconds by an erbium YAG laser with an output of 350 mJ and at a repetition speed of 10 pps.

Thereafter, the temperature immediately after (10 seconds after) the continuous irradiation at the central portion of the probe bending at 30 ° was measured by using a commercially available contact-type thermometer.

The test was performed at room temperature (23 °C)

<Test result> The test result is shown in Table 1.

The measured temperature in the table is an average value of n = 10.

**[Table 1]**

| | Material name of resin layer | Refractive index | Temperature (°C) |
|---|---|---|---|
| Example 1 | Silicon resin | 1.35 | 23.3 |
| Example 2 | Fluoro-acrylic resin | 1.42 | 23.3 |
| Example 3 | Vinyl acetate resin | 1.46 | 23.7 |
| Comparative example 1 | Polymethyl methacrylate | 1.49 | 26.8 |
| Comparative example 2 | Polyimide resin | 1.52 | 41.9 |
| Comparative example 3 | Polycarbonate resin | 1.59 | 47.7 |

### <Discussion>

As shown in the test result in Table 1, it is considered that, in the probes (Comparative examples 1 to 3) having a refractive index of the resin-coated layer higher than that of the cladding (pure silica glass), the laser light leaks at the site that has been subjected to the bending process and is diffused to the metal-coated layer side.

Conversely, it is considered that, in the probes (Examples 1 to 3) having the refractive index of the resin-coated layer lower than that of the cladding, the laser light does not leak at the side that has been subjected to the bending process, and the diffusion of the laser light to the metal-coated layer is limited.

It is known that the refractive index of the pure silica layer is about 1.463.

Since the refractive index of Comparative example 1 was slightly higher than that of the pure silica, the temperature was confirmed to rise by several °C, which is clearly not preferable.

Example 3 was at the same level as the pure silica in terms of the refractive index.

Therefore, a large degree of temperature rise was not confirmed.

However, on the assumption that the bending angle may be 30 ° or more and that the laser irradiation of higher power may be performed for a long time, it is considered that the margin of the temperature rise may be small.

From the result above, it was determined that the refractive index of the resin-coated layer used in the dental probe of the invention is preferably lower than that of the pure silica layer, and more preferably by 0.04 or more lower than that of the pure silica layer.

Additionally, the presence or absence of the light leakage depends on the difference in the refractive index between the cladding of the optical fiber and the resin-coated layer outside thereof.

Consequently, even when the material of the cladding is not a pure silica layer, the temperature rise can be suppressed in the same manner by setting the refractive index of the resin-coated layer to be lower than that of the cladding.

### INDUSTRIAL APPLICABILITY

According to the present invention, since the resin-coated layer is formed of a polymer compound having a refractive index lower than that of the cladding, the resin-coated layer can suppress the leakage of the laser light caused by bending loss, by functioning as the second cladding in the optical fiber.

As a result, it is possible to prevent a temperature rise in the probe.

### DESCRIPTION OF THE REFERENCE SYMBOLS

10:Dental probe
11 :Probe tip portion
20:Optical fiber
21:Core
22:Cladding
23:Resin-coated layer
24:Metal-coated layer
30:Multi-lumen tube
31:Water flow path
32:Air flow path
33:Duct
40:Laser handpiece

## Claims

1. A dental probe (10) provided in a laser handpiece (40) for guiding laser light transmitted through an optical fiber (20) to a position to be irradiated during dental treatment,
wherein a tip portion (11) of the dental probe comprises a water flow path (31), an air flow path (32), a multi-lumen tube (30) including a duct (33) formed therein into which the optical fiber is to be inserted, and the optical fiber inserted into the duct, the multi-lumen tube (30) being formed of a polymer compound having flexibility;
**characterized in that** the optical fiber comprises a resin-coated layer (23) provided on an outer circumference of a cladding (22), and a metal-coated layer (24) provided on an outer circumference of the resin-coated layer (23) and adapted to hold the shape of the optical fiber which is provided in bending while the optical fiber has deformability, and
the resin-coated layer (23) is formed of a polymer compound having a refractive index lower than that of the cladding (22).

2. The dental probe (10) according to Claim 1,
wherein the refractive index of the resin-coated (23) layer is 0.04 or more lower than a refractive index of pure silica.

3. A method for manufacturing a dental probe (10) provided in a laser handpiece (40) for guiding laser light transmitted through an optical fiber (20) to a position to be irradiated during dental treatment, the method comprising:
providing a multi-lumen tube (30) including a water flow path (31), an air flow path (32), and a duct (33) formed therein into which the optical fiber is to be inserted, the multi-lumen tube (30) being formed of a polymer compound having flexibility;
providing the optical fiber (20) comprising
a resin-coated layer (23) provided on an outer circumference of a cladding (22) and formed of a polymer compound having a refractive index lower than that of the cladding, and
a metal-coated layer (24) provided on an outer circumference of the resin-coated layer (23) and adapted to hold the shape of the optical fiber which is provided in bending while the optical fiber has deformability;
preparing a probe tip portion (11) by inserting the optical fiber (20) into the duct (33) of the multi-lumen tube (30); and
insert molding a probe body portion (12) integrally with the multi-lumen tube (30), in which the water flow path (31) and the air flow path (32) are filled with a detachable reinforcement material, a molten resin is supplied to the circumference of the optical fiber (20) and the multi-lumen tube (30) and is solidified, and an optical fiber path (16) is formed in the probe body portion (12) by using the shape of an exterior surface of the optical fiber (20) as a mold.

4. The method for manufacturing a dental probe (10) according to Claim 3, wherein
the probe body portion (12) is made of a polymer compound and is formed integrally with the multi-lumen tube (30), and
the multi-lumen tube (30) is exposed through each of two notches (14, 15) provided on a lateral surface of the probe body portion (12), and thin portions (34, 35) of the multi-lumen tube (30) are cut and opened to form inlets (37, 38) of the water flow path (31) and the air flow path (32).

5. The method for manufacturing a dental probe (10) according to Claim 4, wherein
the notches (14, 15) are formed by providing fins closing the inlets (37, 38) to an interior surface of a mold used for molding the probe body portion (12) such that the molten resin is not provided to the inlets.

6. The method for manufacturing a dental probe (10) according to Claim 4, wherein
in an end surface (39a) at the opposite to a tip surface (39) of the multi-lumen tube (30), the water flow path (31) and the air flow path (32) are closed in the probe body portion (12).

## Patentansprüche

1. Zahnsonde (10), die in einem Laser-Handstück (40) vorgesehen ist, um durch eine optische Faser (20) übertragenes Laserlicht zu einer während der Zahnbehandlung zu bestrahlenden Position zu führen,
wobei ein Spitzenabschnitt (11) der Zahnsonde einen Wasserströmungsweg (31), einen Luftströmungsweg (32), einen Multi-Lumen Schlauch (30) mit einem darin ausgebildeten Kanal (33), in den die optische Faser einzusetzen ist, und die in den Kanal eingesetzte optische Faser umfasst, wobei der Multi-Lumen Schlauch aus einer Polymerverbindung besteht, die biegsam ist;
**dadurch gekennzeichnet, dass** die optische Faser eine auf einem Außenumfang einer Ummantelung (22) aufgetragene Harzschicht (23) sowie eine auf einem Außenumfang der Harzschicht (23) aufgetragene Metallschicht aufweist, die ausgelegt ist, die Form der optischen Faser in ihrem gebogenen Zustand zu halten, während die optische Faser verformbar ist, und
die aufgetragene Harzschicht (23) aus einer Polymerverbindung besteht, deren Brechungsindex niedriger ist als jener der Ummantelung (22).

2. Zahnsonde (10) nach Anspruch 1,
wobei der Brechungsindex der aufgetragenen Harzschicht (23) um 0,04 oder mehr niedriger ist als ein Brechungsindex von reinem Siliciumdioxid.

3. Verfahren zur Herstellung einer Zahnsonde (10), die in einem Laser-Handstück (40) vorgesehen ist, um durch eine optische Faser (20) übertragenes Laserlicht zu einer während der Zahnbehandlung zu bestrahlenden Position zu führen, wobei das Verfahren folgende Schritte aufweist:
Bereitstellen eines Multi-Lumen Schlauches (30) mit einem Wasserströmungsweg (31), einem Luftströmungsweg (32) und einem darin ausgebildeten Kanal (33), in den die optische Faser einzusetzen ist, wobei der Multi-Lumen Schlauch (30) aus einer Polymerverbindung besteht, die biegsam ist;
Bereitstellen der optischen Faser (20), aufweisend,
eine auf einem Außenumfang einer Ummantelung (22) aufgetragene Harzschicht (23) die aus einer Polymerverbindung besteht, deren Brechungsindex niedriger ist als jener der Ummantelung, und
eine auf einem Außenumfang der aufgetragenen Harzschicht (23) aufgetragene Metallschicht (24), die dazu ausgelegt ist, die Form der optischen Faser in ihrem gebogenen Zustand zu halten, während die optische Faser verformbar ist;
Herstellen eines Sondenspitzenabschnitts (11), indem die optische Faser (20) in den Kanal (33) des Multi-Lumen Schlauches (30) eingesetzt wird; und
Umspritzen eines Sondenkörperabschnitts (12) in einem Stück mit dem Multi-Lumen Schlauch (30), wobei der Wasserströmungsweg (31) und der Luftströmungsweg (32) mit einem abnehmbaren Verstärkungsmaterial gefüllt werden, ein geschmolzenes Harz dem Umfang der optischen Faser (20) und dem Multi-Lumen Schlauch (30) zugeführt und verfestigt wird, und bei dem in dem Sondenkörperabschnitt (12) unter Verwendung der Form einer Außenseite der optischen Faser (20) als Form ein Lichtleitweg (16) gebildet wird.

4. Verfahren zur Herstellung einer Zahnsonde (10) nach Anspruch 3, wobei
der Sondenkörperabschnitt (12) aus einer Polymerverbindung besteht und in einem Stück mit dem Multi-Lumen Schlauch (30) hergestellt ist, und
der Multi-Lumen Schlauch (30) durch jede von zwei Kerben (14, 15) auf einer Seitenfläche des Sondenkörperabschnitts (12) freiliegt und dünne Abschnitte (34, 35) des Multi-Lumen Schlauches (30) eingeschnitten und geöffnet werden, um Einlässe (37, 38) des Wasserströmungswegs (31) und des Luftströmungswegs (32) zu bilden.

5. Verfahren zur Herstellung einer Zahnsonde (10) nach Anspruch 4, wobei
die Kerben (14, 15) durch Bereitstellung von Graten gebildet werden, welche die Einlässe (37, 38) zur Innenseite einer Form hin verschließen, die zum Formen des Sondenkörperabschnitts (12) verwendet wird, sodass das geschmolzene Harz nicht zu den Einlässen gelangt.

6. Verfahren zur Herstellung einer Zahnsonde (10) nach Anspruch 4, wobei
in einer zu einer Spitzenfläche (39) des Multi-Lumen Schlauches (30) entgegengesetzten Endfläche (39a) der Wasserströmungsweg (31) und der Luftströmungsweg (32) in dem Sondenkörperabschnitt (12) verschlossen werden.

## Revendications

1. Sonde dentaire (10) fournie dans une pièce à main laser (40) pour guider une lumière laser transmise à travers une fibre optique (20) jusqu'à une position à exposer durant un traitement dentaire,
dans laquelle une partie pointe (11) de la sonde dentaire comprend une trajectoire d'écoulement d'eau (31), une trajectoire d'écoulement d'air (32), un tube à lumières multiples (30) comportant un conduit (33) formé à l'intérieur de celui-ci dans lequel la fibre optique doit être insérée, et la fibre optique insérée dans le conduit, le tube à lumières multiples (30) étant formé d'un composé polymère ayant une flexibilité ; **caractérisée en ce que** la fibre optique comprend une couche revêtue de résine (23) fournie sur une circonférence externe d'une gaine (22), et une couche revêtue de métal (24) fournie sur une circonférence externe de la couche revêtue de résine (23) et adaptée à maintenir la forme de la fibre optique qui est fournie en flexion alors que la fibre optique a une déformabilité, et
la couche revêtue de résine (23) est formée d'un composé polymère ayant un indice de réfraction inférieur à celui de la gaine (22).

2. Sonde dentaire (10) selon la revendication 1,
dans laquelle l'indice de réfraction de la couche revêtue de résine (23) est inférieur de 0,04 ou plus à un indice de réfraction d'une silice pure.

3. Procédé de fabrication d'une sonde dentaire (10) fournie dans une pièce à main laser (40) pour guider une lumière laser transmise à travers une fibre optique (20) jusqu'à une position à exposer durant un traitement dentaire, le procédé comprenant :
la fourniture d'un tube à lumières multiples (30) comprenant une trajectoire d'écoulement d'eau (31), une trajectoire d'écoulement d'air (32), et un conduit (33) formé à l'intérieur de celui-ci dans lequel la fibre optique doit être insérée, le tube à lumières multiples (30) étant formé d'un composé polymère ayant une flexibilité ;
la fourniture de la fibre optique (20) comprenant une couche revêtue de résine (23) fournie sur une circonférence externe d'une gaine (22) et formée d'un composé polymère ayant un indice de réfraction inférieur à celui de la gaine, et
une couche revêtue de métal (24) fournie sur une circonférence externe de la couche revêtue de résine (23) et adaptée à maintenir la forme de la fibre optique qui est fournie lors de la flexion alors que la fibre optique a une déformabilité ;
la préparation d'une partie pointe de sonde (11) par insertion de la fibre optique (20) dans le conduit (33) du tube à lumières multiples (30) ; et
le moulage par insertion d'une partie corps de sonde (12) d'un seul tenant avec le tube à lumières multiples (30), dans lequel la trajectoire d'écoulement d'eau (31) et la trajectoire d'écoulement d'air (32) sont remplies d'un matériau de renforcement détachable, une résine fondue est fournie sur la circonférence de la fibre optique (20) et le tube à lumières multiples (30) et est solidifiée, et une trajectoire de fibre optique (16) est formée dans la partie corps de sonde (12) en utilisant la forme d'une surface extérieure de la fibre optique (20) en tant que moule.

4. Procédé de fabrication d'une sonde dentaire (10) selon la revendication 3, dans lequel
la partie corps de sonde (12) est formée d'un composé polymère et est formée d'un seul tenant avec le tube à lumières multiples (30), et
le tube à lumières multiples (30) est exposé à travers chacune de deux encoches (14, 15) fournies sur une surface latérale de la partie corps de sonde (12), et des parties fines (34, 35) du tube à lumières multiples (30) sont coupées et ouvertes pour former des entrées (37, 38) de la trajectoire d'écoulement d'eau (31) et de la trajectoire d'écoulement d'air (32).

5. Procédé de fabrication d'une sonde dentaire (10) selon la revendication 4, dans lequel
les encoches (14, 15) sont formées par fourniture d'ailettes fermant les entrées (37, 38) vers une surface intérieure d'un moule utilisé pour mouler la partie corps de sonde (12) de telle sorte que la résine fondue ne soit pas fournie aux entrées.

6. Procédé de fabrication d'une sonde dentaire (10) selon la revendication 4, dans lequel
dans une surface d'extrémité (39a) à l'opposé d'une surface de pointe (39) du tube à lumières multiples (30), la trajectoire d'écoulement d'eau (31) et la trajectoire d'écoulement d'air (32) sont fermées dans la partie corps de sonde (12).
